# EUROPEAN PATENT APPLICATION

(11) **EP 1 245 227 A1**
(43) Date of publication of application: **02.10.2002**
(21) Application number: 01108252.6
(22) Date of filing: 31.03.2001
(51) Int. Cl.: A61K 9/20, A61K 9/50

(54) **A pharmaceutical tablet system that floats in the stomach for programmed release of active substance and process of producing buoyant material contained in same**

(71) Applicant: JAGOTEC AG, 4132 Muttenz (CH)
(72) Inventor: Quinton, Jacques, 68640 Waldighofen (FR); Grenier, Pascal, Dr., 68510 Kappelen (FR); Ricchi, Elisabeth, 75019 Paris (FR); Vergnault, Guy, Dr., 68300 Saint Louis (FR)
(74) Representative: Köver, François

(57) **Abstract**

At least part of the pharmaceutical tablet system is made up of buoyant material comprising at least one low density excipient, a hydrophobic material made up of at least one excipient, and a powdered material made up of at least one finely divided filler. The hydrophobic material has a bulk density ≤ 1 and is preferably a pharmaceutically acceptable fatty and/or waxy substance. The powdered material preferably has a loose powder density ≤ 1.

The buoyant material contains voids that are smaller than the grains of the powdered material and may be interstices formed between the grains and sealed off by the hydrophobic material or micropores within the hydrophobic material. It may comprise pharmaceutically active substances and/or carriers. Preferably, it is produced by spray-coating the powdered material with the hydrophobic material, preferably under vigorous stirring; granulating the sprayed material; and compressing the granulated material.

The pharmaceutical tablet system is capable of reliable prolonged floating on gastric fluid in a patient's stomach.

## Description

### TECHNICAL FIELD OF THE INVENTION

This invention concerns a pharmaceutical tablet system capable of prolonged floating on gastric fluid in a patient's stomach so as to bring about therein a programmed release of active substance contained in the tablet system, wherein at least part of the tablet system is made up of buoyant material comprised of at least one low density excipient. This invention also concerns a process of producing buoyant material of said pharmaceutical tablet system.

### BACKGROUND ART

For an overall view of the field of the art to which the invention pertains, reference may be made for instance to Moës, A. J., "Gastroretentive Dosage Forms", Critical Reviews in Therapeutic Drug Carrier Systems 10(2):143-195 (1993).

Pharmaceutical forms having a long time of residence in the stomach are of great interest not only because they allow to treat locally the patient's stomach wall and more particularly the patient's gastric mucous membrane, but also and above all because they allow to release active substance in the vicinity of the patient's duodenum, which is a location of the gastro-intestinal tract where a great many active substances are best absorbed.

There are several approaches for bringing about a long (also called "prolonged") time of residence in the stomach.

A system can be formulated so as to adhere to the gastric mucous membrane (cf. for instance US-A-5213794, US-A-5571533, WO-A-93/24124, WO-A-98/42311, WO-A-98/52547). A major drawback of such adhering systems resides in the difficulty to make them stick to the patient's gastric mucous membrane because the latter is continually undergoing changes and replacement processes. Also, the peristalsis i.e. the strong contractions that take place at the stomach wall make it difficult for pharmaceutical forms to adhere thereto. Currently used pharmaceutical forms designed to adhere e.g. onto nasal or buccal surfaces need to be pressed onto these surfaces at application time, which is not possible with pharmaceutical forms that would be designed to adhere onto the pa-tient's gastric mucous membrane. Moreover, such would-be systems could easily get stuck prematurely in the patient's esophagus.

A system can also be formulated to have a high apparent density that, following ingestion, will cause the system to settle in the stomach at the lower portion of the antrum (cf. for instance US-A-4193985, US-A-5374430). However, the movement of substances contained in the stomach towards the lower portion of the antrum participates in the natural sequence of events of the gastric discharge and hence, pharmaceutical forms formulated so as to settle in the antrum are likely to pass the patient's pylorus either with the bolus (during the digestion process) or together with undigested debris (in the time interval between two successive digestion processes). Thus, to secure the gastroretention of systems formulated so as to have a high apparent density the latter systems must additionally be given some properties that will promote the gastroretention. Indeed, in EP-A-526862 a granulate is disclosed that not only has a high density but also is given muco-adhesive properties.

A system can also be formulated so as to grow in the stomach, following ingestion, to a size large enough to hinder the system from passing the patient's pylorus even when the latter is open. A great many of these systems are either folded at ingestion time and made to unfold and open out in the stomach following ingestion (cf. for instance EP-A-202159, US-A-4735804, US-A-4758436, US-A-4767627, US-A-5002772) or they are made to swell in the stomach, following ingestion, for example as a result of gelling (cf. for instance US-A-4434153, US-A-5651985) or carbon dioxide emission (cf. for instance US-A-4996058, WO-A-98/31341). However, systems formulated to swell could easily pass the patient's pylorus during the latency period that runs from ingestion time until the system has grown to a sufficient size for the gastroretention mechanism to become effective. On the other hand, systems formulated to unfold and open out in the stomach might well be retained permanently in the stomach or even in the esophagus, due to premature activation of the deployment mechanism. Each of such failure cases will cause severe secondary effects.

A system can also be formulated with agents that delay or slow down the transit through the stomach, such as lipid-based vehicles (for instance, fatty acids) or depressors of the central nervous system (for instance, serotonine antagonists). These agents bring about a reduction of the stomach motility, which in turn slows down the gastric discharge. Such a way of bringing about gastroretention is most often used in association with other ways (cf. for instance WO-A-97/47285). However, as systems that bring about a reduction of the stomach motility interfere with the whole mechanism of gastric discharge, they are likely to cause (or worsen already existing) digestion problems. Furthermore, the use of a serotonine antagonist has to comply with pertaining regulations.

A system can also be formulated to float on the content of the stomach.

Such floating systems may be embodied as dense matrices that undergo gelling in the stomach following ingestion, which causes them to swell and hence, reduces their density (cf. for instance US-A-4126672, US-A-4140755, US-A-4167558, US-A-5360793, WO-A-96/29054, discussed below in closer detail); or such floating systems may be embodied as film-coated systems that undergo carbon dioxide emission in the stomach following ingestion, which causes them to foam (an effect that may be understood as a special type of swelling) and hence, reduces their density (cf. for instance US-A-4101650, US-A-4844905, WO-A-98/47506, discussed below in closer detail); or such floating systems may already have a density before ingestion that is sufficiently low to keep them floating in the stomach following ingestion (cf. for instance JP-A-3-101615, US-A-3976764, US-A-4702918, US-A-4814178, US-A-5198229, US-A-5232704, US-A-5288506, US-A-5626876, discussed below in closer detail). More particularly:

US-A-4126672 discloses a system made up of a powder that comprises the active substance, one or more hydrocolloids and optionally a fatty substance. This powder is placed in a capsule. Upon permeation of the gastric fluid through the capsule, the powder undergoes gelling that makes it swell while gaining cohesion and eventually become a gelatinous mass that takes the form of the capsule. In the meantime the capsule gets progressively dissolved, so that the gelatinous mass eventually is relieved of the capsule and begins to float on the content of the stomach, capable of bringing about a slow and prolonged release of the active substance.

WO-A-96/29054 discloses a system that comprises pectin, a compound containing calcium ions, and a carbon dioxide emitting agent. Following ingestion, this system undergoes gelling and at the same time carbon dioxide is emitted that remains trapped in the gelatinous mass. This mechanism builds up a gelatinous layer that will float on the content of the stomach, capable of bringing about a slow and prolonged release of the active substance while also providing a physical barrier against gastroesophageal reflux.

US-A-5360793 discloses a system similar to the above described one, which system comprises xanthan gum, a stabilised hydroxyaluminohexitol and a carbon dioxide emitting agent.

US-A-4140755 discloses a system made up of two layers, each of which comprises active substance. The one layer will bring about an immediate release of the active substance following ingestion. The other layer is formulated with a hydrocolloid that eventually allows the system to float on the content of the stomach following ingestion, due to the formation of a gelatinous mass that will also bring about a slow and prolonged release of the active substance. In the disclosure it is recommended not to subject the two-layered system to a strong compression during manufacture in order to keep the tablet porous with an apparent density that is lower than unity.

US-A-4167558 discloses a system that only has one hydrocolloid containing layer somewhat similar to the corresponding one mentioned above, which layer will bring about a slow and prolonged release of the active substance.

US-A-4844905 discloses a system having a core that comprises active substance, preferably as a granulate, and is coated with a layer capable of emitting carbon dioxide, itself coated with an external layer that is permeable to the gastric fluid but impermeable to the carbon dioxide and that is capable of distension. This system will float for about 15 minutes following ingestion, which will bring about a somewhat prolonged release of the active substance.

WO-A-98/47506 discloses a system formulated with at least one hydrocolloid and a carbon dioxide emitting agent. Following ingestion, the hydrocolloid undergoes gelling and carbon dioxide is emitted that remains trapped in the gelatinous mass. Thus, this system will float following ingestion, which makes it capable of bringing about a prolonged release of the active substance.

US-A-4101650 discloses a system having a core containing a carbon dioxide emitting agent. This core is coated with at least one layer containing one or more hydrocolloids. Following ingestion, the gastric fluid will both make the coating layer swell and cause the emission of carbon dioxide, the latter remaining trapped in the core. Thus, such a system will float following ingestion, which makes it capable of bringing about a prolonged release of the active substance.

US-A-4702918 discloses a system formulated with a capsule that includes a powder comprising the active substance, a compound capable of undergoing gelling and a fatty substance. Heating the fatty substance to fusion and subsequent cooling off brings about cohesion between the powdered active substance and the compound capable of undergoing gelling. The system so obtained has an apparent density that decreases as gelling progresses and eventually becomes lower than unity, which makes it capable of bringing about a prolonged release of the active substance.

However, as said above, all systems formulated to swell or foam, whether by gelling or by trapping carbon dioxide, could easily pass the patient's pylorus before their apparent density has become lower than unity, i.e. during the latency period from ingestion time until the system has undergone sufficient gelling or carbon dioxide trapping for the floating capability and gastroretention mechanism to become effective.

US-A-5288506 discloses a system formulated with one internal phase that comprises active substance and one emulsified external phase comprised of a hydrophobic substance, a hydrophilic substance and a surfactant. The internal phase is granulated with the emulsified external phase. The granules so obtained have an apparent density that is lower than unity, which makes the system capable of bringing about a prolonged release of the active substance.

US-A-5198229 discloses a system having three chambers, a first one from which a prolonged release of the active substance will take place, a second one that allows the system to float on the content of the stomach, and a third one that allows the density of the system to increase when the dissolution progresses, which will cause the system to sink by the end of the dissolution and eventually be evacuated from the stomach. Such a system is very complex and difficult to manufacture on a large scale.

US-A-5232704 discloses a system made up of a gelatine capsule that includes a unit made up of two non-compressed layers. One of the layers is formulated with a matrix that contains the active substance and brings about a prolonged release thereof, the other layer contains a hydrocolloid and allows the system to float by swelling. This system has an apparent density that is lower than unity and decreases further upon contact with the gastric fluid, due to gelling of the hydrocolloid. Also, upon contact with the gastric fluid the capsule will progressively dissolve and the two layers will then swell and stick together. However, the cohesion attained between the two layers is weak and hence, due to the peristalsis that takes place at the stomach, the two layers are likely to separate, which will allow the layer that contains the active substance to sink and eventually be evacuated from the stomach (this latter case is more particularly illustrated in one of the disclosed examples, cf. Table 2, #10).

US-A-3976764 discloses a hollow system (for instance a capsule) coated with a polymer film that contains the active substance. This system has an apparent density that is lower than unity, and the polymer film will bring about a prolonged release of the active substance.

US-A-4814178 discloses a system made up of a tablet that has a low hardness and is formulated with a low proportion of hydrocolloid (2 to 6.5 percent by weight). The hydrocolloid is dissolved in hot water; the solution is then cooled down to a temperature that, however, is kept higher than the gelling temperature of the hydrocolloid; the active substance is then incorporated; and the mixture is then poured into moulds. Upon further cooling a gel will be formed, and finally the resulting tablets are dried. This system has an apparent density that is lower than unity, and it will undergo gelling upon contact with the gastric fluid, which will bring about a prolonged release of the active substance. The major problem posed by this system is that the hardness of the tablet might easily not be sufficient to allow standard drug-manufacturing operations (e.g. packaging) to be performed in usual manner without causing any damage to the tablets. Moreover, the active substance is admixed to the hydrocolloid while the temperature of the solution is kept at about 50-70°C, which could well cause the active substance to become degraded during the manufacturing process of the system.

US-A-5626876 discloses a system made up of a tablet having two portions, one of which is formulated with a matrix that contains the active substance and will bring about a prolonged release thereof, the other portion being inert and hollow so as to give this system an apparent density that is lower than unity. This kind of system may be given many possible configurations. In one instance, the hollow portion can be embodied as a multiplicity of porous particles dispersed in the matrix portion that contains the active substance. In another instance, the hollow portion can be embodied as a core that is completely coated with the matrix portion that contains the active substance.

JP-A-3-101615 discloses a system based on a principle similar to that of the above described one. Hollow granules are made by performing a granulation step on a strongly water-absorbing polymer in the presence of water, then forming a film on the granules, subsequently evaporating the water, and finally coating the granules with a polymer membrane that contains the active substance.

To say nothing of the fact that some of the floating systems mentioned above may have their own severe drawbacks, all of these systems (with the single exception of the above-mentioned US-A-4140755) only bring about a single period of release of active substance (irrespective of the fact that the active substance may actually consist of a mixture of active compounds). As to the system disclosed in the above-mentioned US-A-4140755, this latter system only will bring about an immediate release of active substance followed by a prolonged release of the same active substance.

All the more, none of the floating systems mentioned above is capable of bringing about in the stomach two or more fast releases of active substance separated by respective latency periods during which no release of active substance will take place. Such a capability that has not yet been attained is, however, highly desirable in that it would allow a patient to take one single drug unit form to produce a drug plasma level scheme that can only result at present times from the patient's taking in succession two or more standard-type fast-release drug unit forms at respective predefined time instants separated by respective predefined waiting periods.

It may be noted that fatty and/or waxy substances have been used to obtain tablet systems having a low density.

JP-A-1-016715 discloses a system having a fatty core made up of fats and oils of density ≤ 0.98 and at least one coating layer that contains active substance. Fats and oils that are currently used (alone or in mixture) to confer a density that is lower than unity do not allow tablet production using a compression step of the kind performed in a currently used press, because of feeding and sticking problems: the flow properties of these fats and oils (whether taken as powders or liquids) do not allow to reliably obtain a regular filling of the press moulds, and also, at the compression step these fats and oils stick to the moulding plug and impair the compression.

### SUMMARY OF THE INVENTION

Thus, it is an object of the present invention to make available a pharmaceutical tablet system capable of prolonged floating on the gastric fluid in a patient's stomach under conditions that are safe for the patient, which pharmaceutical tablet system does not have the drawbacks of the floating systems of the background art mentioned above.

It is a further object of the present invention to make available a pharmaceutical tablet system capable of prolonged floating on gastric fluid in a patient's stomach until all of the active substance contained in the pharmaceutical tablet system has been completely released (irrespective of the fact that the active substance may actually consist of a mixture of active compounds), which pharmaceutical tablet system does not have the drawbacks of the floating systems of the background art mentioned above.

It is a still further object of the present invention to make available a pharmaceutical tablet system capable of prolonged floating on gastric fluid in a patient's stomach to bring about therein a programmed release of active substance contained in the tablet system (irrespective of the fact that the active substance may actually consist of a mixture of active compounds), which pharmaceutical tablet system does not have the drawbacks of the floating systems of the background art mentioned above and is capable of reliably bringing about in the stomach a prolonged release of active substance.

It is a still further object of the present invention to make available a pharmaceutical tablet system capable of prolonged floating on gastric fluid in a patient's stomach to bring about therein a programmed release of active substance contained in the tablet system (irrespective of the fact that the active substance may actually consist of a mixture of active compounds), which pharmaceutical tablet system does not have the drawbacks of the floating systems of the background art mentioned above and is capable of reliably bringing about in the stomach two or more fast releases of active substance separated by respective latency periods during which no release of active substance will take place.

To attain these objects and other objects that will become apparent from the description of the invention given below, a pharmaceutical tablet system according to the present invention consists of a pharmaceutical tablet system capable of prolonged floating on gastric fluid in a patient's stomach so as to bring about therein a programmed release of active substance contained in the tablet system, wherein at least part of the tablet system is made up of buoyant material comprised of at least one low density excipient, said buoyant material is comprised of a hydrophobic material made up of an excipient or a mixture of excipients and of a powdered material made up of a finely divided filler or a mixture of finely divided fillers, said hydrophobic material having a bulk density that is about equal to or lower than that of water and said powdered material having a loose powder density that is lower than that of water, and said buoyant material contains voids whose dimensions are essentially smaller than those of powder grains of the powdered material.

The pharmaceutical tablet system of the present invention is capable of reliable prolonged floating on gastric fluid in a patient's stomach, due to the fact that it is not only the apparent overall density of the pharmaceutical tablet system, but also the actual density of the buoyant material, which is lower than that of water. This feature allows to incorporate active substance or substances into the buoyant material and/or into material to be placed on the buoyant material for instance as a coating layer that will include all of the buoyant material or as a layer that will be attached adjacent to, or in the vicinity of, part of the buoyant material, as may be practicable and useful. The resulting geometrical structure of the pharmaceutical tablet system thus produced can be of the substantially concentric type or the dissymetric type, as disclosed (in respect of the geometry, without regard to the nature and content of the core and layers) e.g. in EP-A-210540 or EP-A-788790, respectively.

More particularly, using proper films coated onto the buoyant material, the pharmaceutical tablet system of the present invention will allow to bring about in the stomach two fast releases of active substance separated by a latency period during which no release of active substance will take place, whatever the duration of this latency period, because the system will float on the gastric fluid in the stomach until all of the active substance has been fully released (irrespective of the fact that the active substance may actually consist of a mixture of active compounds). In this case, the system may be constructed in such manner that the external film coating contains active substance, the other film coating is devoid of active substance, and the buoyant material contains active substance and will continue to float until it is completely degraded and disappears.

The above said capacity of the pharmaceutical tablet system of the present invention can easily be extended to more than two fast releases of active substance separated by respective latency periods.

Hence, the pharmaceutical tablet system of the present invention allows a patient to take one single drug unit form to produce a drug plasma level scheme equivalent to that which would result from the patient's taking in succession two or more standard-type fast-release drug unit forms at respective predefined time instants separated by respective predefined waiting periods.

Preferably, in a pharmaceutical tablet system according to the present invention said voids are contained in the buoyant material as interstices formed between powder grains of the powdered material and, preferably, essentially sealed off from each other by virtue of the hydrophobic material, or as micropores included within the hydrophobic material.

Also preferably, said hydrophobic excipient is a pharmaceutically acceptable fatty and/or waxy substance.

Also preferably, said buoyant material also comprises one or more pharmaceutically active substances and/or one or more pharmaceutically acceptable carriers.

Also preferably, said buoyant material is produced by means of a process that includes the following steps: spray-coating the powdered material with the hydrophobic material, preferably under vigorous stirring; granulating the sprayed material; and compressing the granulated material to form the buoyant material.

Also preferably, in a pharmaceutical tablet system according to the present invention said buoyant material is produced by means of the above said process.

More particularly when the pharmaceutical tablet system of the present invention is produced by means of the above said process, it has been found that the buoyant material used therein is well adapted to be compressed in currently used rotary or reciprocating presses without giving rise to any sticking or feeding problems. This finding is quite surprising in view of the difficulties that are encountered when fats and oils are used to obtain a low apparent density as taught in the prior art (e.g. of JP-A-1-016715 quoted above), in particular: unreliable and irregular filling of press moulds, sticking to the moulding plug, impaired compression.

Moreover, it has been found that it is easy to impart the buoyant material appropriate hardness and friability properties that will allow an easy handling of intermediate and final products during subsequent operations such as film coating, packaging etc. This appears to be an important advantage inherent to producing the pharmaceutical tablet system of the present invention according to the above said process.

### BRIEF DESCRIPTION OF THE DRAWINGS

Fig. 1 illustrates an exemplary embodiment of a tablet system according to the present invention with a cylindri cal tablet viewed in a schematic axial section;
Fig. 2 illustrates in vitro release characteristics of the tablet system of Fig. 1.

### DETAILED DESCRIPTION OF THE INVENTION

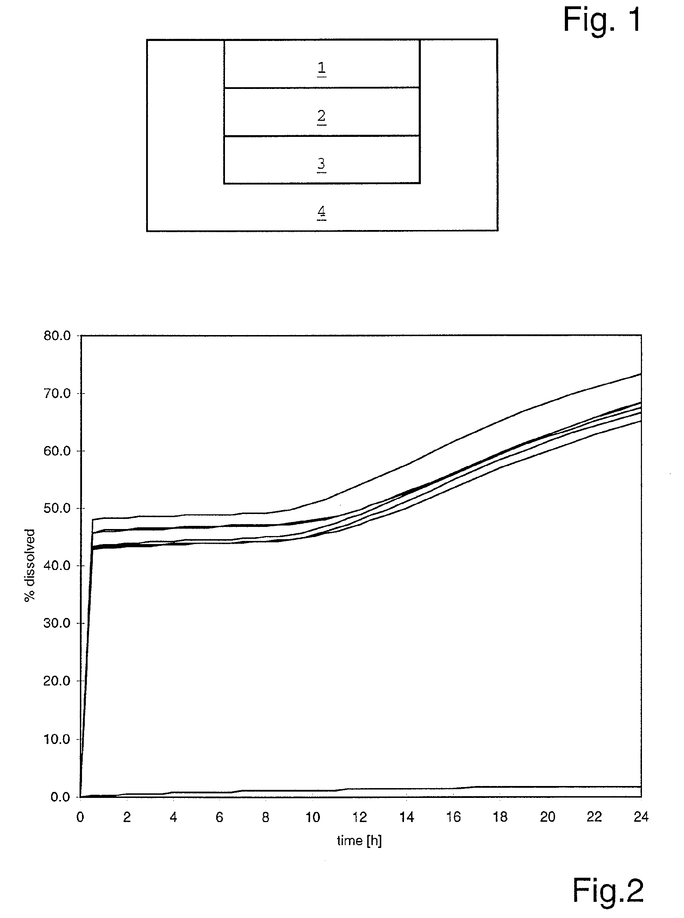

The present invention will now be explained in closer detail with reference to an exemplary tablet system illustrated schematically in Fig. 1 that represents an axial section of a cylindrical tablet.

Generally, the tablet of Fig. 1 has a core that is partially enclosed within an external buoyancy conferring layer made up of buoyant material. The core is a generally cylindrical stack of three layers that are superposed sandwich-like with an intermediate layer 2 located between active layers 1 and 3. These active layers 1 and 3 contain active substance that is, by way of example, diltiazem HCl. The intermediate layer 2 is designed as a barrier layer devoid of active substance. The buoyancy conferring layer 4 is generally shaped as a blind-end hollow cylinder having an axial cylindrical cavity that snugly accommodates the core i.e. the stack of layers 1, 2 and 3.

### PREPARATION

### 1. Preparation of active layers

Active layers i.e. layers containing active substance were prepared, each having a weight of 62.50 mg and the following percentage composition (by weight):

| | |
|---|---|
| diltiazem HCl | 30.00 % |
| lactose (lactose pulvis H2O, 200Mesh) from Paul Brem AG, Switzerland | 59.50 % |
| sodium croscarmellose Ac-Di-Sol ^{(R)}, from FMC Corporation, USA | 5.00 % |
| polyvinylpyrrolidone Plasdone ^{(R)} K29-32, from ISP AG, Switzerland | 4.00 % |
| | |
| magnesium stearate from Merck, Germany | 1.00 % |
| colloidal silica Aerosil^{(R)} 200, from Degussa AG, Hanau, Germany | 0.50 % |
| Total composition | 100.00 % |

Granulate was prepared in an amount appropriate to allow the production of 12000 cores of the type described above i.e. of 24000 active layers.

Proper amounts of Diltiazem HCl, lactose, sodium croscarmellose and polyvinylpyrolidone were placed in a mixer (from Stephan, Switzerland) and mixed therein. Subsequently the homogeneous mixture was wetted with demineralised water and then further mixed, a process known in the art as a "wet massing" step.

The paste so obtained was dried in a fluidised air bed drier (type Niro-Aeromatic Strea I, 60°C inlet air temperature, from Aeromatic-Fielder AG, Switzerland). The resulting dried mass was then sized through a sieve granulator (type Frewitt GLA, from Frewitt Fabrique de Machines SA, Switzerland) with a sieve of 0.8 mm aperture, which step produced calibrated granulate.

This calibrated granulate was then placed in a cubic mixer (type Erweka, from Mapag Maschinen AG, Switzerland), added with a proper amount of colloidal silica, and mixed for 15 min at 12 rpm. Then, a proper amount of magnesium stearate was added, and mixing was continued for 5 min. This mixture was then used for the compression step as described below.

### 2. Preparation of barrier layers

Barrier layers i.e. layers devoid of active substance were prepared, each having a weight of 100.00 mg and the following percentage composition (by weight):

| | |
|---|---|
| dibasic calcium phosphate from Emcompress (R), Mendell, USA) | 45.00 % |
| lactose (lactose pulvis H2O, 200Mesh) Lactose Fast Flo (R), from Foremost, USA | 20.00 % |
| glyceryl behenate Compritol ^{(R)} 888 ATO, from Gattefossé, France | 25.00 % |
| polyvinylpyrrolidone Plasdone ^{(R)} K29-32, from ISP AG, Switzerland | 8.40 % |
| yellow ferric oxide Sicovit ^{(R)} Yellow 10E172, from Bascom AG, Switzerland | 0.10 % |
| magnesium stearate from Merck, Germany | 1.00 % |
| colloidal silica Aerosil^{(R)} 200, from Degussa AG, Hanau, Germany | 0.50 % |
| Total composition | 100.00 % |

Granulate was prepared in an amount appropriate to allow the production of 15000 cores of the type described above i.e. of 15000 barrier layers.

Proper amounts of dibasic calcium phosphate, lactose, glyceryl behenate, polyvinylpyrolidone and yellow ferric oxide were placed in a mixer (from Stephan, Switzerland) and mixed therein. The homogeneous mixture was then wetted with demineralised water and then further mixed in a "wet massing" step.

The paste so obtained was dried in a fluidised air bed drier (type Niro-Aeromatic Strea I, 50°C inlet air temperature, from Aeromatic-Fielder AG, Switzerland). The resulting dried mass was then sized through a sieve granulator (type Frewitt GLA, from Frewitt Fabrique de Machines SA, Switzerland) with a sieve of 0.8 mm aperture, which step produced calibrated granulate.

This calibrated granulate was then placed in a cubic mixer (type Erweka, from Mapag Maschinen AG, Switzerland), added with a proper amount of colloidal silica, and mixed for 15 min at 12 rpm. Then, a proper amount of magnesium stearate was added, and mixing was continued for 5 min. This mixture was then used for the compression step as described below.

### 3. Preparation of buoyant material

Buoyant material was prepared, having the following percentage composition (by weight):

| | |
|---|---|
| hydrogenated castor oil Cutina HR ^{(R)}, from Impag AG, Switzerland | 70.00 % |
| magnesium aluminometasilicate Neusilin UFL ^{(R)}, from Gustav Parmentier, Germany | 12.25 % |
| microcrystalline cellulose Avicel ^{(R)} pH 101, from Selectchemie AG, Switzerland | 12.25 % |
| gelatin from Merck, Germany | 5.00 % |
| magnesium stearate from Merck, Germany | 0.50 % |
| Total composition | 100.00 % |

Granulate was prepared in an amount appropriate to allow the production of 1000 barrier layers each having a weight of 500.00 mg appropriate to enclose 1000 cores so as to manufacture 1000 tablets.

Proper amounts of hydrogenated castor oil, magnesium aluminometasilicate and cellulose microcrystalline were placed in a high shear mixer (type Niro-Fielder PP1, from Aeromatic-Fielder AG, Switzerland). The homogeneous mixture was then wetted with a gelatin solution made up of gelatin previously dissolved in demineralised water and then further mixed in a "wet massing" step.

The paste so obtained was dried in a fluidised air bed drier (type Niro-Aeromatic Strea I, 50°C inlet air temperature, from Aeromatic-Fielder AG, Switzerland). The resulting dried mass was then sized through a sieve granulator (type Frewitt GLA, from Frewitt Fabrique de Machines SA, Switzerland) with a sieve of 0.8 mm aperture, which step produced calibrated granulate.

This calibrated granulate was then placed in a cubic mixer (type Erweka, from Mapag Maschinen AG, Switzerland), added with a proper amount of colloidal silica, and mixed for 10 min at 12 rpm. This mixture was then used for the compression step as described below.

### 4. Preparation of cores

Cores were prepared by means of a rotating three layer press (type Manesty LP39, from Keyser Mackay, Switzerland) equipped with circular convex punches having a diameter of 7.0 mm, operating on the granulates prepared as described above with bulk active layer material in the first and third filling hoppers and bulk barrier layer material in the second filling hopper.

### 5. Application of buoyancy conferring layers onto cores

The cores previously prepared as described above were press-coated with the buoyant material prepared as described above by means of a single punch machine (type Korsch, from Korsch Maschinenfabrik, Germany) equipped with dies and circular convex punches having a diameter of 13.0 mm. The die was filled with an exact quantity of the buoyant material and then the core was placed manually in the die and centred. Subsequently, the compression step was then performed.

The resulting tablets had a thickness of 7.10 mm and a hardness of about 75N.

### RESULTS

To determine the in vitro release characteristics of the tablets described above, a standard equipment was used as defined and described in United States Pharmacopoeia USP XXIII, chapter 711, page 1792, paragraph "Apparatus 2". This equipment had a stirring paddle comprised of a blade and a shaft and was operated at 100 rpm. Dissolution was investigated at 37°C in 600 ml of a dissolution medium made up of 0.1M acetate buffer of pH 4.5. The release of the active substance (diltiazem HCl) was monitored by UV spectrophotometry at 278 nm for 6 individual samples and additionally, as a reference, for the dissolution medium taken alone i.e. devoid of any tablet material.

The results are illustrated in Fig.2 as respective time profile diagrams for the 6 tablet samples and the reference. The reference diagram showed that the dissolution medium taken alone i.e. devoid of any tablet material did not bias the results or generate any artifacts. The in vitro release characteristics of all 6 tablets appeared to form a well grouped family that was well separated from the reference characteristic which appeared in the lowest part of the diagram.

In each instance, the following was observed on the in vitro release characteristics:

The first release of active substance takes place within a release period of less than a one hour duration.

The no-release period appears as a well-defined time interval observed between the end of the first release and the start of the second release, having a duration of more than 8 hours in each instance.

The second release of active substance is observed to produce a controlled release.

During the course of the dissolution the tablet system was monitored visually and observed to remain buoyant for the whole duration of the experiment.

## Claims

1. A pharmaceutical tablet system capable of prolonged floating on gastric fluid in a patient's stomach so as to bring about therein a programmed release of active substance contained in the tablet system, wherein at least part of the tablet system is made up of buoyant material comprised of at least one low density excipient,
**characterized in that**
said buoyant material is comprised of a hydrophobic material made up of an excipient or a mixture of excipients and of a powdered material made up of a finely divided filler or a mixture of finely divided fillers, said hydrophobic material having a bulk density that is about equal to or lower than that of water and said powdered material having a loose powder density that is lower than that of water, and
said buoyant material contains voids whose dimensions are essentially smaller than those of powder grains of the powdered material.

2. A pharmaceutical tablet system according to claim 1, **characterized in that** said voids are contained in the buoyant material as interstices formed between powder grains of the powdered material.

3. A pharmaceutical tablet system according to claim 2, **characterized in that** said voids are essentially sealed off from each other by virtue of the hydrophobic material.

4. A pharmaceutical tablet system according to claim 1, **characterized in that** said voids are contained in the buoyant material as micropores included within the hydrophobic material.

5. A pharmaceutical tablet system according to claim 1, **characterized in that** said hydrophobic excipient is a pharmaceutically acceptable fatty and/or waxy substance.

6. A pharmaceutical tablet system according to claim 1, **characterized in that** said buoyant material also comprises one or more pharmaceutically active substances.

7. A pharmaceutical tablet system according to claim 1, **characterized in that** said buoyant material also comprises one or more pharmaceutically acceptable carriers.

8. A pharmaceutical tablet system according to claim 1, **characterized in that** said buoyant material is produced by means of a process that includes the following steps:
spray-coating the powdered material with the hydrophobic material, preferably under vigorous stirring;
granulating the sprayed material; and
compressing the granulated material to form the buoyant material.

9. A process of producing buoyant material of a pharmaceutical tablet system according to claim 1, **characterized by** the following steps:
spray-coating the powdered material with the hydrophobic material, preferably under vigorous stirring;
granulating the sprayed material; and
compressing the granulated material to form the buoyant material.
